(19) Europäisches Patentamt
European Patent Office
Office européen des brevets

(11) Veröffentlichungsnummer : **0 473 541 A2**

(12) **EUROPÄISCHE PATENTANMELDUNG**

(21) Anmeldenummer : **91810623.8**

(22) Anmeldetag : **08.08.91**

(51) Int. Cl.[5] : **B01D 61/24,** B01D 71/56, B01D 71/64, C07C 51/42

(30) Priorität : **17.08.90 CH 2675/90**

(43) Veröffentlichungstag der Anmeldung : **04.03.92 Patentblatt 92/10**

(84) Benannte Vertragsstaaten : **CH DE FR GB IT LI**

(71) Anmelder : **CIBA-GEIGY AG**
**Klybeckstrasse 141**
**CH-4002 Basel (CH)**

(72) Erfinder : **Berger, Joseph, Dr.**
**Sperrstrasse 40/18**
**W-4057 Basel (CH)**
Erfinder : **Wernet, Wolfgang, Dr.**
**The Rokko Apt. 301, 3-14-12**
**Shinohara-Kita-muchi**
**Nada-ku, 650 Kobe (JP)**

(54) **Anreicherungs- oder Trennverfahren, Copolyamide und Copolyimidamide und deren Verwendung.**

(57) Gemische von Salzen organischer Carbonsäuren und nicht salzartigen organischen Verbindungen, gelöst in einem $C_1$-$C_4$-Alkanol, können mit einer semipermeablen Membran aus einem Copolyamid oder Copolyimidamid, das (a) einen ersten aromatischen Diaminrest und (b) einen zweiten aromatischen Diaminrest mit $-SO_3M$-Gruppen enthält, wobei M für $H^{\oplus}$, ein ein- bis dreiwertiges Metallkation oder ein Ammoniumkation darstellt, getrennt oder angereichert werden. Sofern der erste Diaminrest $C_1$-$C_4$-Alkylgruppen in den o-Stellungen zu den Amingruppen enthält, sind die Copolymeren strahlungsempfindlich und können zur Herstellung von Schutzschichten oder Reliefabbildungen verwendet werden, wobei die Entwicklung in einem wässrig-basischen Medium vorgenommen wird.

EP 0 473 541 A2

Die vorliegende Erfindung betrifft ein Verfahren zur Anreicherung oder Trennung von Salzen organischer Carbonsäuren von nicht salzartigen organischen Verbindungen, bei dem man eine Lösung dieser Salze und Verbindungen in einem niedermolekularen Alkanol mit einer Seite einer semipermeablen Membran aus einem Sulfonsäuregruppen enthaltenden Copolyamid oder Copolyimidamid in Berührung bringt, wobei sich auf der Gegenseite der Membran das reine niedermolekulare Alkanol befindet; aromatische Copolyamide und Copolyimidamide aus zwei aromatischen Diaminen, von denen eines Sulfonsäuregruppen enthält und ein Verfahren zu deren Herstellung, sowie ein beschichtetes Material und ein Verfahren zur Herstellung von Schutzschichten oder Reliefabbildungen unter Verwendung dieses Materials.

In CA 110:25827c (1989) sind Copolyamide aus (1) gegebenenfalls kernalkylierten Diphenylsulfondiaminen, (2) aromatischen gegebenenfalls versalzte Sulfonsäuregruppen enthaltenden aromatischen Diaminen und aromatischen Dicarbonsäuren beschrieben. Semipermeable Membranen weisen bei der Entsalzung wässriger Salzlösungen durch Umkehrosmose ein verbessertes Salzrückhaltevermögen aus.

Es wurde überraschend gefunden, dass mit Membranen aus Copolyamiden oder Copolyimidamiden, in denen ein Aminrest gegebenenfalls versalzte Sulfonsäuregruppen enthält, auch Mischungen aus Salzen organischer Carbonsäuren und nicht salzartigen organischen Verbindungen in Form von Lösungen in niedermolekularen Alkanolen angereichert oder getrennt werden können. Bei Verwendung von Copolyamiden mit Diphenylsulfondiaminresten permeiert überraschend bevorzugter das Salz.

Ein Gegenstand der Erfindung ist ein Verfahren zum Anreichern oder Trennen von Salzen organischer Carbonsäuren von nicht salzartigen organischen Verbindungen mit einer semipermeablen Membran, dadurch gekennzeichnet, dass man eine Lösung der Salze und organischen Verbindungen in einem unsubstituierten oder mit $C_1$-$C_3$-Alkoxy substituierten $C_1$-$C_4$-Alkanol, Mischungen solcher Alkanole oder Mischungen solcher Alkanole mit Ethern mit einer Seite der Membran in Berührung bringt, sich auf der Gegenseite der Membran das reine Lösungsmittel befindet, und die Membran aus einem Copolyamid oder Copolyimidamid aus (a) mindestens einem aromatischen Dicarbonsäurerest mit 8 bis 20 C-Atomen und/oder (b) mindestens einem trivalenten aromatischen Tricarbonsäurerest mit 9 bis 20 C-Atomen, die je unsubstituiert oder mit Halogen, Nitro, $C_1$-$C_4$-Alkyl oder $C_1$-$C_4$-Alkoxy substituiert sind, (c) mindestens einem ersten divalenten und/oder trivalenten ein- oder zweikernigen aromatischen Diaminrest und (d) mindestens einem zweiten divalenten und/oder trivalenten und mindestens eine -$SO_3M$-Gruppe enthaltenden aromatischen ein- oder zweikernigen Diaminrest, die je 6 bis 18 C-Atome enthalten und je unsubstituiert oder mit Halogen oder $C_1$-$C_4$-Alkyl substituiert sind, und M $H^{\oplus}$, ein ein- bis dreiwertiges Metallkation, $NH_4^{\oplus}$ oder ein organisches Ammoniumkation mit 1 bis 30 C-Atomen bedeutet.

Die Konzentration der Salze und organischen Verbindungen beträgt bevorzugt 0,0001 bis 10, besonders 0,001 bis 5 und insbesondere 0,001 bis 3 Gewichtsprozent, bezogen auf die Lösung.

Die Dicke der Membran kann z.B. 5 bis 300 µm, bevorzugt 20 bis 200 µm betragen.

M in der Gruppe $SO_3M$ kann als Ammoniumkation $NH_4^{\oplus}$ oder ein Ammoniumkation eines primären, sekundären oder tertiären offenkettigen Amins mit bevorzugt 1 bis 24, besonders 1 bis 16 C-Atomen bedeuten, oder ein Ammoniumkation eines monocyclischen oder bicyclischen sekundären oder tertiären oder eines tricyclischen tertiären Amins mit bevorzugt 4 bis 12 C-Atomen sein.

M in der Bedeutung eines Metallkations kann ein ein- bis dreiwertiges Kation der Metalle der Haupt- und Nebengruppen, der Uebergangsmetalle und der Edelmetalle sein. Bevorzugt sind ein- oder zweiwertige Metallkationen. Beispiele für Metalle sind Li, Na, K, Rb, Cs, Mg, Ca, Sr, Ba, In, Sn, Pb, Cu, Ag, Au, Zn, Cd, Hg, Cr, Mo, Mn, Fe, Co, Ni, Rn, Rh, Pd, Ir, Pt, Sb, Bi, sowie die Gruppe der Seltene-Erdmetalle. Bevorzugte Metalle sind die Alkali- und Erdalkalimetalle, Cu, Ag, Au, Fe, Co, Ni, Zn, Cd und Mn.

In einer bevorzugten Ausführungsform steht M für $H^{\oplus}$, $NH_4^{\oplus}$, ein Alkalimetallkation oder ein primäres, sekundäres, tertiäres oder quaternäres Ammoniumkation mit 1 bis 24 C-Atomen.

Das als Lösungsmittel verwendete Alkanol enthält 1 bis 4 C-Atome und bevorzugt 1 bis 3 C-Atome. Es kann z.B. mit Methoxy oder Ethoxy substituiert sein. Beispiele sind Methanol, Ethanol, n- und i-Propanol, n-, i- und t-Butanol, Methoxyethanol, Ethoxyethanol, Propoxyethanol, 1-Methoxy-propan-3-ol, 2-Methoxy-propan-1-ol. Bevorzugte Lösungsmittel sind Methanol, Ethanol, 1- oder 2-Propanol und 2-Methoxyethanol. Es können auch Mischungen der Alkanole untereinander oder mit Ethern, z.B. Diethylether oder Ethylenglykoldimethylether verwendet werden.

Bei dem Salz der organischen Carbonsäure kann es sich um Ammonium- oder Metallsalze handeln, z.B. $NH_4^{\oplus}$, einem Ammoniumkation eines primären, sekundären oder tertiären Amins mit insgesamt 1 bis 20 C-Atomen, Alkalimetallsalze und Erdalkalimetallsalze. Bevorzugt sind Alkalimetallsalze und Ammoniumsalze. Besonders bevorzugt sind $NH_4^{\oplus}$- und $Li^{\oplus}$-Salze.

Bei der organischen Carbonsäure kann es sich z.B. um Mono-, Di-, Tri- oder Tetracarbonsäuren handeln. Bevorzugt sind aliphatische, cycloaliphatische, aromatische und heterocyclische oder heteroaromatische Monocarbonsäuren, die 1 bis 18 C-Atome, vorzugsweise 1 bis 12 C-Atome enthalten.

Eine bevorzugte Ausführungsform ist dadurch gekennzeichnet, dass die organische Säure der Formel (A)

$R_a$-X-COOH (A)

entspricht, worin X eine direkte Bindung, $C_1$-$C_4$-Alkylen, $C_2$-$C_4$-Alkyliden oder $C_2$-$C_4$-Alkenylen bedeutet, $R_3$ für H, $C_1$-$C_{12}$-Alkyl, $C_2$-$C_{12}$-Alkenyl, $C_2$-$C_{12}$-Alkinyl, $C_3$-$C_{12}$-Cycloalkyl, $C_3$-$C_{12}$-Cycloalkenyl, $C_6$-$C_{16}$-Aryl, $C_3$-$C_{12}$-Heterocycloalkyl, $C_3$-$C_{12}$-Heterocycloalkenyl, $C_6$-$C_{16}$-Heteroaryl steht, die unsubstituiert oder mit -OH, -SH, -CN, $-NO_2$, Halogen, $C_1$-$C_6$-Alkyl, $C_1$-$C_6$-Alkoxy, $C_1$-$C_6$-Alkylthio, $C_1$-$C_6$-Alkyl-Y- mit Y gleich -CO-, -SO-, $-SO_2-$, -CO-O-, -O-CO-, -CO-$NR_bR_c$-, -$NR_bR_c$-CO- substituiert sind, und $R_b$ und $R_c$ unabhängig voneinander H, $C_1$-$C_6$-Alkyl, $C_2$-$C_4$-Hydroxyalkyl oder $R_b$ und $R_c$ zusammen Tetramethylen, Pentamethylen oder 3-Oxapentyl-1,4-en darstellen.

Beispiele für $R_a$ als Alkyl, das linear oder verzweigt sein kann, sind Methyl, Ethyl, n- und i-Propyl, n- und i-Butyl, Pentyl, Hexyl, Heptyl, Octyl, Nonyl, Decyl, Undecyl und Dodecyl.

Beispiele für $R_a$ als Alkenyl, das linear oder verzweigt sein kann, sind Vinyl, Crotonyl, Allyl, But-1-en-1-yl, But-1-en-2-yl, But-1-en-3-yl, But-1-en-4-yl, But-2-en-1-yl, But-2-en-2-yl, But-2-en-4-yl, Pentenyl, Hexenyl, Heptenyl, Octenyl, Nonenyl, Decenyl, Undecenyl und Dodecenyl.

Beispiele für $R_a$ als Alkinyl, das linear oder verzweigt sein kann, sind Ethinyl, Prop-2-in-1-yl, Prop-2-in-3-yl, Butinyl, Pentinyl, Hexinyl, Heptinyl, Octinyl, Decinyl und Dodecinyl.

$R_a$ enthält als Cycloalkyl und Cycloalkenyl bevorzugt 4-8 C-Atome, besonders 5 oder 6 C-Atome. Beispiele sind Cyclopropyl und Cyclopropenyl, Cyclobutyl und Cyclobutenyl, Cyclopentyl und Cyclopentenyl, Cyclohexyl und Cyclohexenyl, Cycloheptyl und Cycloheptenyl, Cyclooctyl und Cyclooctenyl.

$R_a$ enthält als Aryl bevorzugt 6 bis 12 C-Atome. Einige Beispiele sind Phenyl, Biphenyl und Naphthyl.

$R_a$ enthält als Heterocycloalkyl und Heterocycloalkenyl bevorzugt 4 bis 8, besonders 4 bis 6 Ring-C-Atome. Bevorzugte Heteroatome sind solche aus der Gruppe O, S und $NR_d$, worin $R_c$ H, $C_1$-$C_6$-Alkyl oder $C_1$-$C_7$-Acyl bedeutet. $R_a$ enthält als Heteroaryl bevorzugt 4 bis 11 Ring-C-Atome und bevorzugt Heteroatome aus der Gruppe O, S und -N=. Einige Beispiele für Heterocyclen sind Pyrrolidin, Tetrahydrofuran, Tetrahydrothiophen, Pyrrolin, Dihydrofuran, Dihydrothiophen, Indan, Dihydrocumaron, Dihydrobenzothiophen, Carbazol, Dibenzofuran, Dibenzothiophen, Pyrazolidin, Imidazolidin, Pyrazolin, Imidazolin, Benzimidazolidin, Oxazolidin, Oxazolin, Thiazolidin, Thiazolin, Isooxazolidin, Isooxazolin, Isothiazolidin, Isothiazolin, Benzoxazolidin, Benzisooxazolidin, Benzthiazolidin, 1,2,3- oder 1,2,4-Triazolidin, 1,2,3- oder 1,2,4-Triazolin, 1,2,3- oder 1,2,4-Oxazolidin oder -Oxazolin, Piperidin, Di- und Tetrahydropyridin, Dihydro- und Tetrahydropyran, Di- und Tetrahydrothiopyran, Piperazin, Dehydropiperazin, Morpholin, Thiomorpholin, 1,3- und 1,4-Dioxan, 1,4-Dithian, Azepan, 1,3-Dioxolan, 1,3-Dithiolan, Pyrrol, Indol, Imidazol, Benzimidazol, Furan, Thiophen, Benzofuran, Benzothiophen, Carbazol, Dibenzofuran, Dibenzothiophen, Oxazol, Isooxazol, Thiazol, Isothiazol, Benzoxazol, Benzothiazol, Pyridin, Pyrimidin, Pyrazin, Pyridazin, Chinolin, Isochinolin, Acridin, Chromen, Chroman, Pyran, Thiapyran, Phenazin, Phenoxazin, Phenolthiazin, Purin.

Beispiele für X in Formel A sind Methylen, Ethylen, 1,2- oder 1,3-Propylen, 1,2-, 1,3- oder 1,4-Butylen, Ethyliden, 1,1- oder 2,2-Propyliden, 1,1- oder 2,2-Butyliden, Ethenylen, Prop-1-en-1,3- oder -1,2- oder -2,3-ylen.

Beispiele für $R_b$ und $R_c$ als Alkyl und Hydroxyalkyl sind Methyl, Ethyl, n- oder i-Propyl, n-, i- oder t-Butyl, n-Pentyl, n-Hexyl, 2-Hydroxyethyl, 2- oder 3-Hydroxypropyl, 2-, 3- oder 4-Hydroxybutyl. Beispiele für $R_c$ als Acyl sind Acetyl, Propionyl und Phenacyl.

Eine bevorzugte Ausführungsforme ist dadurch gekennzeichnet, dass es sich bei dem Salz um $Li^{\oplus}$- oder $NH_4^{\oplus}$-Salze von Carbonsäuren aus der Gruppe Furan-2-carbonsäure, Benzoesäure, Methylbenzoesäure, Phenylessigsäure, Zimtsäure, Sorbinsäure oder $C_2$-$C_8$-Alkancarbonsäuren handelt.

Die nicht salzartige organische Verbindung enthält bevorzugt 2 bis 20, besonders 2 bis 16 und insbesondere 2 bis 12 C-Atome. Insbesondere ist die organische Verbindung ein Ester einer organischen Monocarbonsäure mit insgesamt 2 bis 16 C-Atomen, ein Ether mit 2 bis 12 C-Atomen, ein Keton mit 3 bis 16 C-Atomen oder ein Alkohol mit 5 bis 16 C-Atomen.

Eine bevorzugte Ausführungsform ist dadurch gekennzeichnet, dass die organische Verbindung ein $C_1$-$C_6$-Alkylester von Furan-2-carbonsäure, Benzoesäure, Methylbenzoesäure, Phenylessigsäure, Zimtsäure, Sorbinsäure oder $C_2$-$C_8$-Alkancarbonsäuren; ein $C_5$-$C_{12}$-Alkanol oder Benzylalkohol; ein dialiphatisches Keton mit 3 bis 10 C-Atomen, ein $C_1$-$C_6$-Alkyl-phenylketon oder Diphenylketon; oder ein dialiphatischer Ether mit 2 bis 8 C-Atomen, $C_1$-$C_6$-Alkyl-phenylether oder Diphenylether ist.

Die Membran kann zur Durchführung der erfindungsgemässen Verfahren in verschiedenen Formen ausgebildet und in Trennmodulen üblicher Bauart eingebaut sein. So kann man z.B. Flachmembranen oder asymmetrische Membranen zu Zwei- oder Mehrkammersystemen kombinieren. Man kann auch schlauchförmige Membranen oder Hohlfasern verwenden, die im allgemeinen in Form von Bündeln eingesetzt werden. Zur Erhöhung der mechanischen Stabilität können die Membranen auf ein Stützgerüst aufgebracht werden. Die Herstellung der Membranen erfolgt nach bekannten Verfahren.

Das erfindungsgemässe Verfahren wird im allgemeinen bei Raumtemperatur durchgeführt. Zur Erzielung ausreichender Fliessgeschwindigkeit ist es vorteilhaft, auf der Seite der Lösung einen erhöhten Druck einzustellen. Der Druck beträgt bevorzugt 1 bis 10 MPa, besonders bevorzugt 1 bis 6 MPa. In einer besonderen Ausführungsform wird das Verfahren nach dem Gegenstromprinzip durchgeführt.

Das erfindungsgemässe Verfahren kann zum Beispiel als Anreicherungs- oder Reinigungsverfahren oder zur Rückgewinnung bzw. Abtrennung von Reaktionskomponenten oder Nebenprodukten aus Reaktionsrückständen oder Reaktionsgemischen eingesetzt werden oder zur Isolierung bzw. Reinigung von Zwischenprodukten verwendet werden, besonders wenn es sich um thermisch labile Substanzen handelt.

Bei den aromatischen Dicarbonsäurresten (a) kann es sich um Reste von ein- oder zweikernigen Aryldicarbonsäuren handeln, die bevorzugt insgesamt 8 bis 16 C-Atome enthalten, und die unsubstituiert oder substituiert sind mit bevorzugt F, Cl, Br oder $NO_2$. Bei den aromatischen Tricarbonsäureresten (b) kann es sich um Reste von ein- oder zweikernigen Aryltricarbonsäuren handeln, die bevorzugt 9 bis 17 C-Atome enthalten, und die unsubstituiert oder mit bevorzugt F, Cl, Br oder $NO_2$ substituiert sind.

Die Diaminreste (c) und (d) sind bevorzugt ein- oder zweikernige Arylreste mit bevorzugt 6 bis 14 C-Atome, die unsubstituiert oder mit bevorzugt F, Cl, Br, Methyl oder Ethyl substituiert sind.

Das Copolyamid oder Copolyimidamid enthält bevorzugt 60 bis 95 Mol-%, besonders 75 bis 95 Mol-% Diaminreste (c) und 5 bis 40 Mol-%, besonders 5 bis 25 Mol-% Diaminreste (d), bezogen auf die Diaminreste. Die inhärente Viskosität der Copolyamide, Copolyamidimide oder der Mischpolymere kann 0,2 bis 3,0 dl/g, bevorzugt 0,3 bis 2,0 dl/g und besonders bevorzugt 0,3 bis 1,2 dl/g betragen.

Eine bevorzugte Ausführungsform des erfindungsgemässen Verfahrens ist dadurch gekennzeichnet, dass das Copolyamid und/oder Copolyimidamid

a) 60 bis 95 Mol-% mindestens eines wiederkehrenden Strukturelementes der Formeln I und/oder Ia,

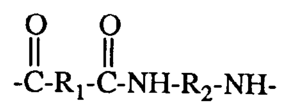

(I),

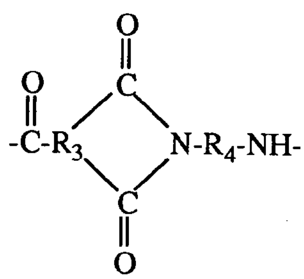

(Ia),

und

b) 5 bis 40 Mol-% mindestens eines wiederkehrenden Strukturelementes der Formeln II und/oder IIa,

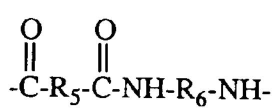

(II),

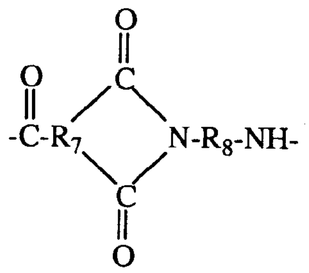

(IIa)

enthalten, bezogen auf die Copolymeren, worin $R_1$ und $R_5$ unabhängig voneinander einen unsubstituierten oder mit Cl, Br, $NO_2$, $C_1$-$C_4$-Alkyl oder $C_1$-$C_4$-Alkoxy substituierten Rest der Formeln

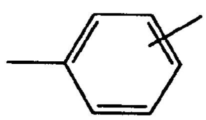 , 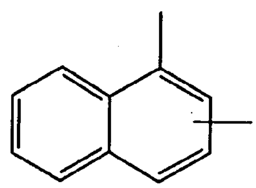 , 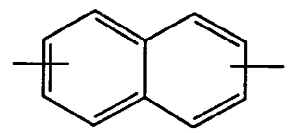 oder

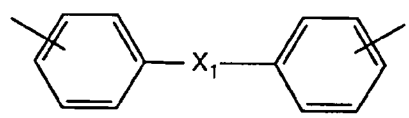

darstellen, worin $X_1$ für eine direkte Bindung, $CH_2$,

$-(CH_2)_2-$, -HC=CH-, $CH_3CH<$, $(CH_3)_2C<$,

-$NR_9$-, -N=N-, -CO-, -O-, -S-, -SO- oder -$SO_2$- steht, und $R_9$ H oder $C_1$-$C_6$-Alkyl bedeutet,
$R_3$ und $R_7$ unabhängig voneinander einen Rest der Formeln

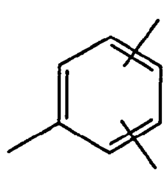 , 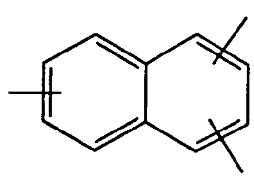 , 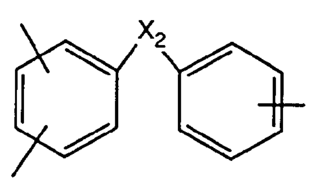

darstellen, worin zwei Bindungen an zwei benachbarte C-Atome gebunden sind, und $X_2$ unabhängig die gleiche Bedeutung wie $X_1$ hat,
$R_2$ und $R_4$ unabhängig voneinander einen unsubstituierten oder mit Cl, Br oder $C_1$-$C_4$-Alkyl substituierten Rest der Formeln

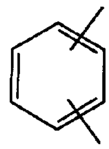 , 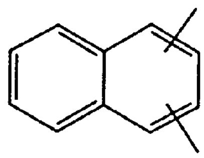 , 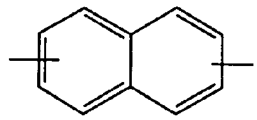 , 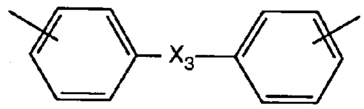

bedeuten, worin $X_3$ unabhängig die gleiche Bedeutung wie $X_1$ hat, und
$R_6$ und $R_8$ unabhängig voneinander einen Rest der Formeln

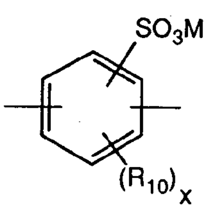 , 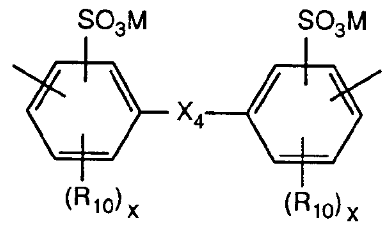

darstellen, worin $X_4$ unabhängig die gleiche Bedeutung wie $X_1$ hat, $R_{10}$ für $C_1$-$C_4$-Alkyl steht, x für 0, 1, 2 oder 3 steht und M $H^{\oplus}$, $NH_4^{\oplus}$, ein Alkalimetallkation oder ein primäres, sekundäres, tertiäres oder quaternäres Ammoniumkation mit 1 bis 24 C-Atomen darstellt.

$X_1$, $X_2$, $X_3$ und $X_4$ stehen unabhängig bevorzugt für eine direkte Bindung $-CH_2-$, -CO-, -S- oder -O-.

Beispiele für aromatische Dicarbonsäuren, von denen sich die Reste $R_1$ und $R_5$ ableiten, sind Terephthalsäure, Isophthalsäure, 4-Nitroisophthalsäure, 4-Chlorisophthalsäure, Tetrabromterephthalsäure, 4,4'-, 3,4'- oder 3,3'-Diphenylmethandicarbonsäure oder -Diphenyldicarbonsäure oder -Diphenyletherdicarbonsäure oder -Diphenylsulfondicarbonsäure oder -Benzophenondicarbonsäure, 1,3-, 1,4-, 2,6- oder 2,7-Naphthalindicarbonsäure. Weitere geeignete aromatische Dicarbonsäuren mit Benzophenoneinheiten sind z.B. in der EP-A-0198798 beschrieben.

Bevorzugte Dicarbonsäuren sind Terephthal- und Isophthalsäure, die mit F, Cl, Br oder $NO_2$ substituiert sein können, 4,4'-Benzophenondicarbonsäure, 4,4'-Diphenyldicarbonsäure, 4,4'-Diphenylmethandicarbonsäure, 4,4'-Diphenyletherdicarbonsäure und 4,4'-Diphenylsulfondicarbonsäure.

Beispiele für aromatische Tricarbonsäuren, von denen sich $R_3$ und $R_7$ ableiten, sind Trimellitsäure, 1,6,7- oder 2,6,7-Naphthalintricarbonsäure, 3,4,4'-Diphenyltricarbonsäure oder -Diphenylmethantricarbonsäure oder -Diphenylethertricarbonsäure oder -Diphenylthioethertricarbonsäure oder -Diphenylsulfontricarbonsäure oder -Benzophenontricarbonsäure. Bevorzugte Tricarbonsäure ist Trimellitsäure.

Beispiele für aromatische Diamine, von denen sich $R_2$ und $R_4$ ableiten, sind in der EP-A-0 138 768 beschrieben. Als Beispiele seien genannt: m- oder p-Phenylendiamin, 4-Chlor-m-phenylendiamin, 3-Chlor-p-phenylendiamin, mono- bis tetramethylsubstituierte m- oder p-Phenylendiamine; 1,3-, 2,6- oder 2,7-Naphthylendiamine, die mit Cl, Methyl oder Ethyl substituiert sein können; oder Diamine der Formel B

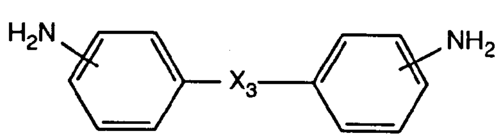 (B),

worin die $NH_2$-Gruppen in m- oder p-Stellung zur $X_3$-Gruppe stehen, $X_3$ eine direkte Bindung, $-CH_2-$,

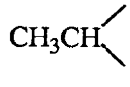 , 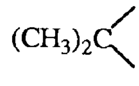 ,

-O- , -S- , $-SO_2-$ oder -CO- bedeutet, und die Benzolreste mit vorzugsweise Methyl oder Ethyl substituiert sein

können, bevorzugt in einer oder beiden o-Stellungen zur $NH_2$-Gruppe. Bevorzugte Diamine sind m- und p-Phenylendiamin, 4-Chlor-m-phenylendimain, 4,4′-Diamino-3,3′-dimethyl-diphenylmethan, 4,4′-Diamino-3,3′,5,5′-tetramethyl-diphenylmethan, 4,4′-Diamino-2,2′,6,6'-tetramethyl-diphenylmethan.

Bei den Diaminen, von denen sich die Reste $R_6$ und $R_6$ ableiten, kann es sich um die gleichen Diamine wie für $R_2$ und $R_4$ handeln, wobei diese zusätzlich eine $SO_3M$-Gruppe enthalten, oder ein Substituent durch eine $SO_3M$-Gruppe ersetzt ist. Die $SO_3M$-Gruppe ist bevorzugt in den m-Stellungen zu den Aminogruppen gebunden. Einige Beispiele sind (nur als Sulfonsäuren angegeben): 3,5-Diaminobenzol-1-sulfonsäure, 3,5-Diamino-2-methyl- oder -4-methylbenzol-1-sulfonsäure, 3,5-Diamino-2,4-dimethyl- oder -2,6-dimethylbenzolsulfonsäure, 3,5-Diamino-2,4,6-trimethylbenzol-1-sulfonsäure, 3,6- oder 3,7-Diaminonaphthalin-1-sulfonsäure, 3,3′-Diaminodiphenyl-5,5′-disulfonsäure, 3,3′-Diamino-4,4′-dimethyl-diphenyl-5,5′-disulfonsäure, 3,3′-Diamino-2,2′,4,4′-tetramethyl-diphenyl-5,5′-disulfonsäure, 4,4′-Diamino-diphenyl-2,2′-disulfonsäure, 4,4′-Diamino-3,3′- oder -5,5′-dimethyl-diphenyl-2,2′-disulfonsäure, 4,4′-Diamino-3,3′,5,5′-tetramethyl-diphenyl-2,2′-disulfonsäure sowie entsprechende Diphenylmethan-, Diphenylether-, Diphenylthioether-, Diphenylsulfon- und Benzophenondiamino-disulfonsäuren.

Eine bevorzugte Ausführungsform des erfindungsgemässen Verfahrens ist dadurch gekennzeichnet, dass es sich um ein Copolyamid mit Strukturelementen der Formeln I und II handelt, und $X_1$ und $X_4$ unabhängig voneinander eine direkte Bindung, $-CH_2-$ , -CO- , -O- oder -S- bedeuten.

Eine andere bevorzugte Ausführungsform ist dadurch gekennzeichnet, dass in den Strukturelementen der Formeln I und II des Copolyamids $R_1$ und $R_5$ unabhängig voneinander für unsubstituiertes oder mit $-NO_2$-, -Cl oder -Br substituiertes m- und/oder p-Phenylen steht, $R_2$ unsubstituiertes oder mit -Cl, -Br, Methyl oder Ethyl substituiertes Phenylen oder in beiden o-Stellungen zu den Aminogruppen mit Methyl und/oder Ethyl substituiertes 3,3′- oder 4,4′-Diphenylmethandiyl bedeutet, und $R_6$ einen Rest der Formeln

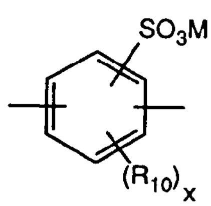

,

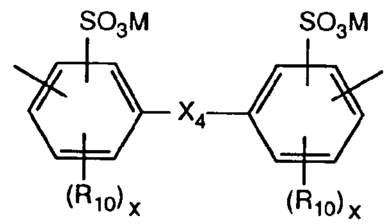

darstellt, worin $X_4$ eine direkte Bindung oder $-CH_2-$ bedeutet, $R_{10}$ für Methyl oder Ethyl steht, x für 1, 2 oder 3 steht, und M $H^{\oplus}$, $NH_4^{\oplus}$, $Li^{\oplus}$, $Na^{\oplus}$, $K^{\oplus}$, $Rb^{\oplus}$, $Cs^{\oplus}$ oder quaternäres Ammonium mit 4 bis 16 C-Atomen bedeutet.

Eine besonders bevorzugte Ausführungsform ist dadurch gekennzeichnet, dass $R_6$ ein Rest der Formel

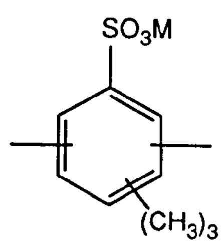

ist, worin M $H^{\oplus}$, $NH_4^{\oplus}$, $Li^{\oplus}$, $Na^{\oplus}$, $K^{\oplus}$, $Rb^{\oplus}$, $Cs^{\oplus}$ oder quaternäres Ammonium mit 4 bis 16 C-Atomen bedeutet.

Die Herstellung der Copolyamide und Copolyimidamide erfolgt für die im erfindungsgemässen Verfahren verwendeten Membranen nach bekannten Verfahren. Die Monomeren sind entweder bekannt oder können nach bekannten Verfahren hergestellt werden. Die Herstellung der Membranen erfolgt nach bekannten Verfahren, z.B. mittels dem Foliengiessverfahren.

In der EP-A-0 138 768 und der EP-A-0 198 798 sind strahlungsempfindliche Polyamide und Polyimidamide beschrieben, die Diaminreste mit Alkylgruppen in den o-Stellungen zu den Aminogruppen und Reste von Benzophenongruppen enthaltenden Dicarbonsäuren enthalten. Diese Polymeren sind nur in polaren und aprotischen organischen Lösungsmitteln löslich, und nach der Bestrahlung unter einer Photomaske kann die Entwicklung nur mit organischen Lösungsmitteln vorgenommen werden, was unter ökologischen Gesichtspunkten nachteilig ist.

Es wurde gefunden, dass Copolyamide und Copolyimidamide aus aromatischen Dicarbonsäuren bzw. Tricarbonsäuren und aromatischen Diaminen mit Alkylgruppen in den o-Stellungen zu den Aminogruppen und aromatischen Diaminen mit $SO_3M$-Gruppen überraschend strahlungsempfindlich, in polaren und aprotischen Lösungsmitteln löslich und in wässrig-basischen Medien löslich und entwickelbar sind. Die mechanische Stabilität und Lösungsmittelbeständigkeit von Membranen für das erfindungsgemässe Trennverfahren aus solchen Polymeren kann durch Bestrahlung verbessert werden.

Ein weiterer Gegenstand der Erfindung sind Copolymere aus der Gruppe der Copolyamide und Copolyimidamide mit einer inhärenten Viskosität von 0,2 bis 3,0 dl/g, gemessen bei 25°C in einer Lösung von 0,5 Gew.-% Copolymer in N-Methylpyrrolidon, die enthalten, bezogen auf das Copolymer:

a) 10 bis 95 Mol-% mindestens eines wiederkehrenden Strukturelements der Formeln III und/oder IIIa

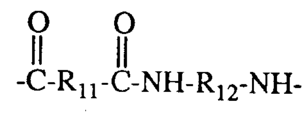

(III),

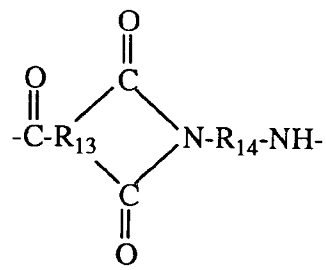

(IIIa),

und

b) 5 bis 90 Mol-% mindestens eines wiederkehrenden Strukturelements der Formeln IV und/oder IVa

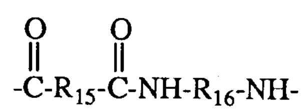

(IV),

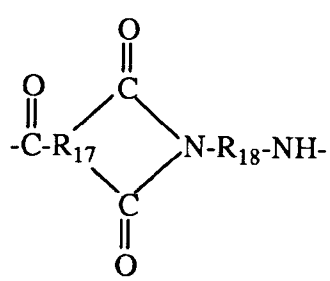

(IVa)

worin

$R_{11}$ und $R_{15}$ unabhängig voneinander der divalente Rest einer ein- oder zweikernigen aromatischen Dicarbonsäure mit 8 bis 20 C-Atomen bedeuten, die je unsubstituiert oder mit Halogen, Nitro, $C_1$-$C_4$-Alkyl oder $C_1$-$C_4$-Alkoxy substituiert sind,

$R_{13}$ und $R_{17}$ unabhängig voneinander der trivalente Rest einer ein- oder zweikernigen aromatischen Tricarbonsäure mit 9 bis 20 C-Atomen darstellen, die je unsubstituiert oder mit Halogen oder Nitro substituiert sind,

$R_{12}$ und $R_{14}$ unabhängig voneinander einen Rest der Formeln

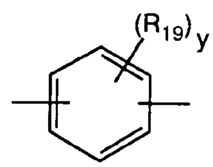

oder

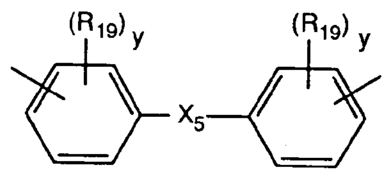

darstellen,
worin $R_{19}$ $C_1$-$C_4$-Alkyl bedeutet, $X_5$ eine direkte Bindung, $-CH_2-$,

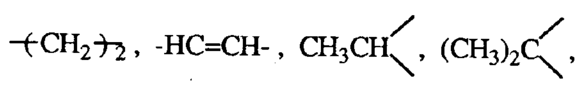

$-NR_9-$, -N=N-, -CO-, -O- oder -S- darstellt und $R_9$ für H oder $C_1$-$C_6$-Alkyl steht, y für 0, 1, 2, 3 oder 4 steht und
$R_{16}$ und $R_{18}$ unabhängig voneinander einen Rest der Formeln

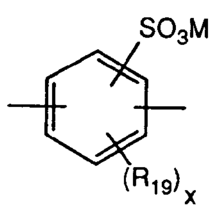

oder

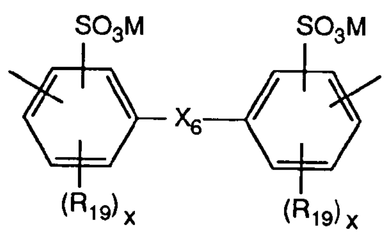

darstellen, worin $R_{19}$ $C_1$-$C_4$-Alkyl bedeutet, x für 0, 1, 2 oder 3 steht, $X_6$ eine direkte Bindung, $-CH_2-$,

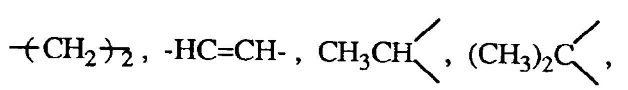

$-NR_9-$, -N=N-, -CO-, -O-, -S-, -SO- oder $-SO_2-$ bedeutet und $R_9$ für H oder $C_1$-$C_6$-Alkyl steht, und M $H^{\oplus}$, $NH_4^{\oplus}$, ein einwertiges Metallkation oder ein organisches Ammoniumkation mit 1 bis 30 C-Atomen bedeutet, mit der Massgabe, dass in mindestens einem der Reste $R_{12}$, $R_{14}$, $R_{16}$ und $R_{18}$ mindestens zwei Reste $R_{19}$ in den Orthostellungen der freien Bindungen gebunden sind.

Bevorzugt steht y für 2, 3 oder 4. Ferner steht x bevorzugt für 2 oder 3. $R_{19}$ bedeutet bevorzugt Ethyl und besonders bevorzugt Methyl. In einer bevorzugten Ausführungsform stehen y für 2, 3 oder 4 und x für 2 oder 3 und je 2 Reste $R_{19}$ sind bevorzugt in den beiden o-Stellungen zur freien Bindung gebunden.

Die Copolymeren enthalten bevorzugt 60 bis 95 Mol-% Strukturelemente der Formeln III und/oder IIIa und 5 bis 40 Mol-% Strukturelemente der Formeln IV und/oder IVa.

Geeignete aromatische Di- und Tricarbonsäure sind zuvor erwähnt worden. Bevorzugte Copolymeren sind solche, worin $R_{11}$ und $R_{15}$ unabhängig voneinander einen unsubstituierten oder mit Cl, Br oder $NO_2$ substituierten Rest der Formeln

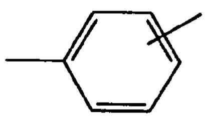 , 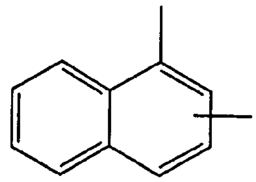 , 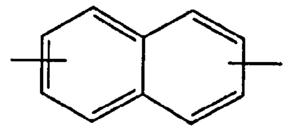 oder

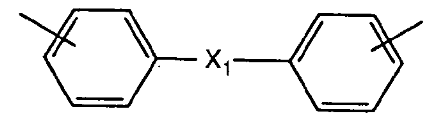

darstellen, worin $X_1$ für eine direkte Bindung, $CH_2$,

$-(CH_2)_2-$, -HC=CH-, 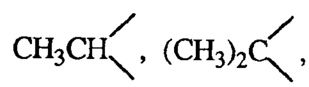 , -

$-NR_9-$, -N=N-, -CO-, -O-, -S-; -SO- oder $-SO_2-$ steht, und $R_9$ H oder $C_1$-$C_6$-Alkyl bedeutet, und $R_{13}$ und $R_{17}$ unabhängig voneinander einen Rest der Formeln

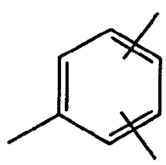 , 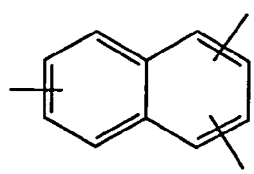 , 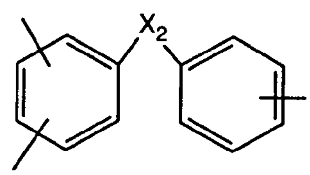

darstellen, worin zwei Bindungen an zwei benachbarte C-Atome gebunden sind, und $X_2$ unabhängig die gleiche Bedeutung wie $X_1$ hat.

Eine bevorzugte Ausführungsform ist dadurch gekennzeichnet, dass $R_{11}$ und $R_{15}$ unabhängig voneinander unsubstituiertes oder mit Cl, Br oder $NO_2$ substituiertes o-, m- oder p-Phenylen oder einen Rest der Formel

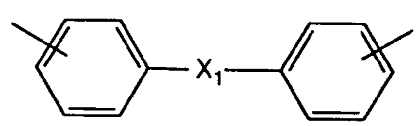

darstellen, worin die freien Bindungen in m- oder p-Stellung zur Gruppe $X_1$ gebunden sind und $X_1$ eine direkte Bindung, $-CH_2-$, -O-, -S-, $-SO_2-$ oder -CO- bedeutet.

M steht bevorzugt für $H^{\oplus}$, $NH_4^{\oplus}$, ein Alkalimetallkation oder ein primäres, sekundäres, tertiäres oder quaternäres Ammoniumkation mit 1 bis 24 C-Atomen.

$R_{19}$ steht bevorzugt für Methyl oder Ethyl.

$R_{12}$ und $R_{14}$ bedeuten bevorzugt unabhängig voneinander einen Rest der Formeln

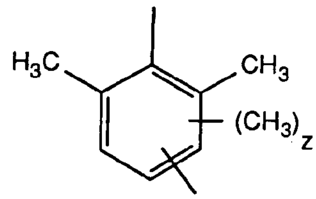 oder 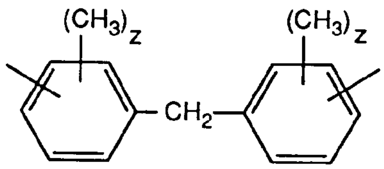,

worin in der ersten Formel z 0, 1 oder 2 ist und die freien Bindungen in m- oder p-Stellung zueinander stehen, und in der zweiten Formel die freien Bindungen in m- oder p-Stellung zur $CH_2$-Gruppe gebunden sind, und die beiden $CH_3$-Gruppen je in den Orthostellungen der freien Bindungen gebunden sind.

$R_{16}$ und $R_{18}$ bedeuten bevorzugt unabhängig voneinander einen Rest der Formeln

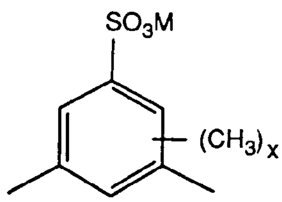 oder 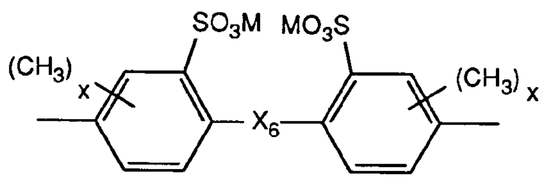,

worin x 0, 1, 2 oder 3 ist, $X_6$ eine direkte Bindung, $-CH_2-$, -O-, -S-, -CO- oder $-SO_2-$ bedeutet und M für $H^{\oplus}$, $NH_4^{\oplus}$, ein Alkalimetallkation oder primäres, sekundäres, tertiäres oder quaternäres Ammonium mit 1 bis 24 C-Atomen steht.

Eine besonders bevorzugte Ausführungsform ist dadurch gekennzeichnet, dass es ein Copolyamid mit wiederkehrenden Strukturelementen der Formeln

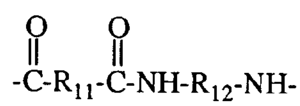 und

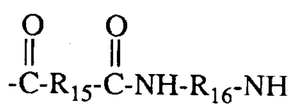

ist,

worin $R_{11}$ und $R_{15}$ unabhängig voneinander unsubstituiertes oder mit Cl, Br oder Nitro substituiertes m- oder p-Phenylen oder

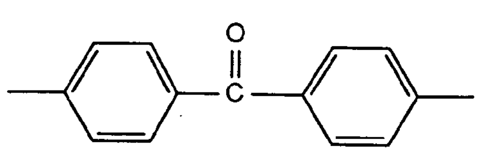

bedeuten,

$R_{12}$ für einen Rest der Formeln

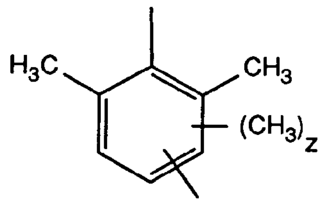 oder 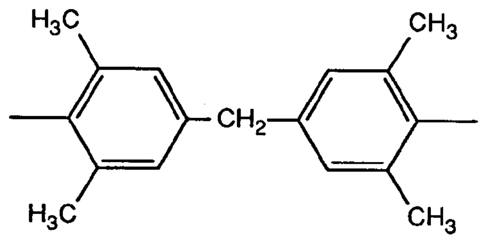

steht, worin z 0, 1 oder 2 ist, und
$R_{16}$ einen Rest der Formel

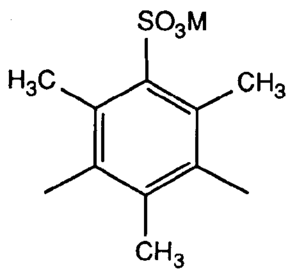

darstellt, worin M für $H^{\oplus}$, $NH_4^{\oplus}$, ein Alkalimetallkation oder primäres, sekundäres, tertiäres oder quaternäres Ammonium mit 1 bis 24 C-Atomen steht.

Ein weiterer Gegenstand der Erfindung ist ein Verfahren zur Herstellung der erfindungsgemässen Copolymere, das dadurch gekennzeichnet ist, dass man, bezogen auf das Copolymer,

a) 50 Mol-% mindestens einer Dicarbonsäure der Formel V und/oder Va

$$\mathrm{HO\overset{\overset{O}{\|}}{C}-R_{11}-\overset{\overset{O}{\|}}{C}OH} \qquad (V)$$

$$\mathrm{HO\overset{\overset{O}{\|}}{C}-R_{15}-\overset{\overset{O}{\|}}{C}OH} \qquad (Va)$$

und/oder mindestens einer Tricarbonsäure der Formeln VI und/oder VIa

$$R_{13}\text{-}(COOH)_3 \quad (VI)$$
$$R_{17}\text{-}(COOH)_3 \quad (VIa)$$

oder deren Polyamid bildenden Derivate mit

b) 5 bis 47,5 Mol-% mindestens eines Diamins der Formeln VII und/oder VIIa

$$H_2N\text{-}R_{12}\text{-}NH_2 \quad (VII)$$
$$H_2N\text{-}R_{14}\text{-}NH_2 \quad (VIIa)$$

und

c) 2,5 bis 45 Mol-% mindestens eines Diamins der Formeln VIII und/oder VIIIa

$$H_2N\text{-}R_{16}\text{-}NH_2 \quad (VIII)$$
$$H_2N\text{-}R_{18}\text{-}NH_2 \quad (VIIIa)$$

umsetzt.

Die im erfindungsgemässen Verfahren verwendeten Monomeren sind bekannt und teilweise käuflich.

Geeignete Polyamid bildende Derivate sind z.B. die Säureanhydride, Säureamide, Säurehalogenide und Säureester. Geeignete Herstellungsverfahren sind zum Beispiel die Lösungsmittelpolykondensation, die

Schmelzkondensation und die Grenzflächenpolykondensation. Geeignete Lösungsmittel sind nachfolgend erwähnt.

Die Reaktionstemperaturen richten sich im wesentlichen nach den Ausgangsmaterialien, Herstellungsverfahren bzw. deren Reaktivität. Sie kann -50 bis 350°C, vorzugsweise 50 bis 300°C betragen. Ferner kann die Polykondensation bei Normaldruck oder Unterdruck durchgeführt werden. Vorteilhaft wird bei der Kondensation entstehendes Wasser, Alkohol oder Amin während des verfahrens aus dem Reaktionsgemisch entfernt bzw. entstehende Halogenwasserstoffe, z.B. HCl oder HBr, durch Zugabe geeigneter Mittel wie tertiäre Amine oder Epoxide gebunden.

Bei den erfindungsgemässen Copolymeren handelt es sich um Polymere, die direkt durch Einwirkung von Strahlung vernetzt werden können. Sie eignen sich zur Herstellung von Folien, Fasern und zur Beschichtung von Substraten, für den Oberflächenschutz oder zur Erzeugung von Reliefabbildungen, wobei durch Bestrahlung die Eigenschaften der Polykondensate modifiziert werden können. Die hochschmelzenden aromatischen Copolymeren werden bevorzugt aus Lösung verarbeitet.

Ein bevorzugter Anwendungsbereich ist die Verwendung zur Beschichtung von Oberflächen und die Herstellung von Reliefabbildungen auf solchen beschichteten Substraten, was ein weiterer Gegenstand vorliegender Erfindung ist. Es ist besonders vorteilhaft, dass zur Erzielung gewünschter Eigenschaften die erfindungsgemässen Copolymere für bestimmte Anforderungen bei der Anwendung durch Auswahl unterschiedlicher Monomerer und/oder durch Abmischung verschiedener Copolymere entsprechend eingestellt werden können. Ein weiterer besonderer Vorteil der erfindungsgemässen Polymeren ist deren Löslichkeit in wässrig-basischen Medien, z.B. in wässrigen Alkalimetallhydroxidlösungen (z.B. NaOH, KOH), oder wässrigen Alkalimetallcarbonaten (z.B. $NaHCO_3$, $Na_2CO_3$, $K_2CO_3$).

Ein weiterer Gegenstand der Erfindung ist ein beschichtetes Material, enthaltend auf einem Träger eine Schicht eines erfindungsgemässen Copolymers, wobei $X_5$ zusätzlich -SO- oder $-SO_2-$ bedeuten kann.

Zur Herstellung des erfindungsgemässen beschichteten Materials löst man ein Copolymer oder Gemische davon zweckmässig in einem geeigneten organischen Lösungsmittel, gegebenenfalls unter Erwärmen. Geeignete Lösungsmittel sind z.B. polare, aprotische Lösungsmittel, die alleine oder in Mischungen aus mindestens zwei Lösungsmitteln verwendet werden können. Beispiele sind: Ether wie Dibutylether, Tetrahydrofuran, Dioxan, Ethylenglykol, Dimethylethylenglykol, Dimethyldiethylenglykol, Diethyldiethylenglykol, Dimethyltriethylenglykol, halogenierte Kohlenwasserstoffe wie Methylenchlorid, Chloroform, 1,2-Dichlorethan, 1,1,1-Trichlorethan, 1,1,2,2-Tetrachlorethan, Carbonsäureester und Lactone wie Essigsäureethylester, Propionsäuremethylester, Benzoesäureethylester, 2-Methoxyethylacetat, γ-Butyrolacton, o-Valerolacton und Pivalolacton, Carbonsäureamide und Lactame wie Formamid, Acetamid, N-Methylformamid, N,N-Dimethylformamid, N,N-Dimethylformamid, N,N-Dimethylacetamid, N,N-Diethylacetamid, γ-Butyrolactam, ε-Caprolactam, N-Methylpyrrolidon, N-Acetylpyrrolidon, N-Methylcaprolactam, Tetramethylharnstoff, Hexamethylphosphorsäuretriamid, Sulfoxide wie Dimethylsulfoxid, Sulfone wie Dimethylsulfon, Diethylsulfon, Trimethylensulfon, Tetramethylensulfon, Trimethylamin, Triethylamin, N-Methylpyrrolidin, N-Methylpiperidin, N-Methylmorpholin, substituierte Benzole wie Chlorbenzol, Nitrobenzol, Phenole oder Kresol.

Ungelöste Anteile können durch Filtration, bevorzugt einer Druckfiltration, entfernt werden. Die Konzentration an Polymer im so erhaltenen Beschichtungsmittel beträgt vorzugsweise nicht mehr als 50 Gew.-%, besonders nicht mehr als 30 Gew.-% und insbesondere nicht mehr als 20 Gew.-%, bezogen auf die Lösung. Die Lösungen sind lagerstabil.

Bei der Herstellung der Lösungen können weitere übliche Zusatzstoffe einverleibt werden, die die Lichtempfindlichkeit nicht negativ beeinflussen. Beispiele hierfür sind Mattierungsmittel, Verlaufmittel, feinteilige Füllstoffe, Flammschutzmittel, optische Aufheller, Antioxidantien, Lichtschutzmittel, Stabilisatoren, Farbstoffe, Pigmente und Haftvermittler. Ferner können auch, falls erwünscht, zusätzlich Sensibilisatoren, wie z.B. Thioxanthonderivate oder Benzophenonderivate einverleibt werden, um die Lichtempfindlichkeit noch weiter zu erhöhen.

Das Beschichtungsmittel kann mittels üblichen Methoden wie Tauch-, Streich- und Sprühverfahren, Schleuder-, Kaskadenguss- und Vorhanggussbeschichtung, auf geeignete Substrate bzw. Trägermaterialien, aufgebracht werden. Geeignete Substrate sind z.B. Kunststoffe, Metalle und Metalllegierungen, Halbmetalle, Halbleiter, Glas, Keramik und andere anorganische Materialien wie z.B. $SiO_2$ und $Si_3N_4$. Danach wird das Lösungsmittel gegebenenfalls durch Erwärmen und gegebenenfalls im Vakuum entfernt. Man erhält klebfreie, trockene und gleichmässige Filme. Die aufgebrachten Filme können je nach Anwendung Schichtdicken bis zu ca. 500 μm und mehr, bevorzugt von 0,5 bis 500 μm und besonders von 1 bis 50 μm aufweisen.

Die strahlungsempfindliche Schicht im erfindungsgemässen Material kann durch Einwirkung von Strahlung vernetzt werden.

Die Photostrukturierung bzw. Photovernetzung kann durch energiereiche Strahlung hervorgerufen werden, z.B. durch Licht insbesondere im UV-Bereich, durch Röntgenstrahlen, Laserlicht, Elektronenstrahlen usw. Das

erfindungsgemässe Material eignet sich hervorragend zur Herstellung von Schutzfilmen, Passivierlacken, und als photographisches Aufzeichnungsmaterial für thermostabile Reliefabbildungen.

Ein weiterer Gegenstand der Erfindung ist diese Verwendung. Anwendungsgebiete sind z.B. Schutz-, Isolier- und Passivierlacke in der Elektrotechnik und Elektronik, Photomasken für die Elektronik, den Textildruck und das graphische Gewerbe Aetzresiste zur Herstellung gedruckter Schaltungen und Druckplatten und integrierter Schaltkreise, Relais für die Herstellung von Röntgenmasken, als Lötstoplack, als Dielektrikum für Mehrlagenschaltungen, als Strukturelement für Flüssigkristallanzeiger.

Die Herstellung von Schutzfilmen erfolgt durch direktes Belichten, wobei sich die Belichtungszeiten im wesentlichen nach den Schichtdicken und der Lichtempfindlichkeit richten.

Die photographische Erzeugung der Reliefstruktur erfolgt durch bildmässige Belichtung durch eine Photomaske, anschliessende Entwicklung unter Entfernung der unbelichteten Anteile mit einem wässrig-basischen Medium, besonders wässrigen Alkalimetallhydroxidlösungen (NaOH, KOH) oder wässrigen Alkalimetallcarbonatlösungen ($Na_2CO_3$, $K_2CO_3$, $KHCO_3$, $NaHCO_3$), wonach gegebenenfalls das erzeugte Bild durch eine thermische Nachbehandlung stabilisiert werden kann.

Ein weiterer Gegenstand der Erfindung ist ein Verfahren zur Herstellung von Schutzschichten oder Reliefabbildungen, dadurch gekennzeichnet, dass man die Schicht des Materials flächenmässig bestrahlt, oder unter einer Photomaske bestrahlt und danach in einem wässrig-basischen Medium durch Lösen der unbestrahlten Anteile entwickelt.

Die Polymerschicht des erfindungsgemässen Materials weist eine für viele Anwendungszwecke ausreichende und teilweise hohe Lichtempfindlichkeit auf und sie kann direkt photovernetzt werden. Die Schutzfilme und Reliefabbildungen zeichnen sich durch gute Haftfestigkeit und thermische, mechanische und chemische Widerstandsfähigkeit aus. Bei thermischen Nachbehandlungen wird nur ein geringer Schwund beobachtet. Ferner können Zusätze zur Erzeugung bzw. Steigerung der Lichtempfindlichkeit vermieden werden. Das Material ist lagerstabil, ist aber vor Lichteinwirkung zu schützen.

Die nachfolgenden Beispiele erläutern die Erfindung näher.

A) Herstellung der Copolymeren

Beispiele A1-A15:

In einem 200 ml Glaskolben mit Rührer, Rückflusskühler, Tropftrichter, Trockenrohr und Kühlbad werden die Diamine in N-Methylpyrrolidon (NMP) gelöst vorgelegt. Die Lösung wird auf 15°C gekühlt und Propylenoxid zugegeben. Dann werden während 15 Minuten bei kräftigem Rühren die Dicarbonsäuredichloride zugetropft. Die Lösung wird weitere 3 Stunden bei 15 bis 20°C gerührt. Darauf wird das Copolymere durch Zugabe von Methanol gefällt, der Niederschlag abfiltriert und im Hochvakuum bei 40°C getrocknet. Weitere Angaben befinden sich in der nachfolgenden Tabelle 1:

Tabelle 1

| Bsp. Nr. | Diamin 1 (mMol) | Diamin 2 (mMol) | 1. Dicarbon-säuredichlorid (mMol) | 2. Dicarbon-säuredichlorid (mMol) | NMP [1] (Menge in g) | PPO [2] (Menge in g) | Inhärente [3] Viskosität (dl/g) |
|---|---|---|---|---|---|---|---|
| A1 | 4,4'-Diamino-3,5,3',5'-tetramethyl-diphenyl-methan (22.5) | 3,5-Diamino-2,4,6-trimethylbenzol-1-sulfonsäure-tetra-butylammoniumsalz (2.5) | Isophthalsäure-dichlorid (7.5) | Terephthal-säuredichlorid (17.5) | 78 | 12 | 0.89 |
| A2 | 4,4'-Diamino-3,5,3',5'-tetramethyl-diphenyl-methan (13.5) | 3,5-Diamino-2,4,6-trimethylbenzol-1-sulfonsäure-tetra-butylammoniumsalz (1.5) | 4-Nitro-iso-phthalsäure-dichlorid (7.5) | Terephthal-säuredichlorid (7.5) | 62 | 8 | 0.69 |
| A3 | 4,4'-Diamino-2,6,2',6'-tetramethyl-diphenyl-methan (12.9) | 3,5-Diamino-2,4,6-trimethylbenzol-1-sulfonsäure-tetra-butylammoniumsalz (1.5) | Isophthalsäure-dichlorid (4.3) | Terephthal-säuredichlorid (10.1) | 75 | 5 | 0.31 |
| A4 | p-Phenylendiamin (16.0) | 3,5-Diamino-2,4,6-trimethylbenzol-1-sulfonsäure-tetra-butylammoniumsalz (4.0) | Isophthalsäure-dichlorid (6.0) | Terephthal-säuredichlorid (14.0) | 120 | 7 | 0.85 |

Tabelle 1 (Fortsetzung)

| Bsp. Nr. | Diamin 1 (mMol) | Diamin 2 (mMol) | 1. Dicarbonsäuredichlorid (mMol) | 2. Dicarbonsäuredichlorid (mMol) | NMP [1] (Menge in g) | PPO [2] (Menge in g) | Inhärente [3] Viskosität (dl/g) |
|---|---|---|---|---|---|---|---|
| A5 | 4-Chloro-m-phenylendiamin (18.0) | 3,5-Diamino-2,4,6-trimethylbenzol-1-sulfonsäure-tetra-butylammoniumsalz (2.0) | Isophthalsäure-dichlorid (6.0) | Terephthal-säuredichlorid (14.0) | 42 | 12 | 0.60 |
| A6 | 4-Chloro-m-phenylendiamin- (13.5) | 3,5-Diamino-2,4,6-trimethylbenzol-1-sulfonsäure-tetra-butylammoniumsalz (1.5) | 4-Nitro-iso-phthalsäure-dichlorid (7.5) | Terephthal-säuredichlorid (7.5) | 72 | 8 | 0.39 |
| A7 | 4,4'-Diamino-trans-stilben-2,2'-disul-fonsäure-di-tetra-butylammonium-salz (2.0) | 4,4'-Diamino-3,3'-di-ethyl-5,5'-dimethyl-diphenylmethan (8.0) | Isophthal-säuredi-chlorid (3.0) | Terephthal-säuredichlorid (7.0) | 80 | 3,5 | 0,690 |

Tabelle 1 (Fortsetzung)

| Bsp. Nr. | Diamin 1 (mMol) | Diamin 2 (mMol) | 1. Dicarbonsäuredichlorid (mMol) | 2. Dicarbonsäuredichlorid (mMol) | NMP [1] (Menge in g) | PPO [2] (Menge in g) | Inhärente [3] Viskosität (dl/g) |
|---|---|---|---|---|---|---|---|
| A8 | 4,4'-Diamino-diphenyl-2,2'-disulfonsäure-di-tetrabutylammonium-salz (0.5) | 4,4'-Diamino-3,3'-di-ethyl-5,5'-dimethyl-diphenylmethan (9.5) | Isophthalsäure-dichlorid (3.0) | Terephthal-säuredichlorid (7.0) | 80 | 3,5 | 0.482 |
| A9 | 4,4'-Diamino-trans-stilben-2,2'-disulfon-säure-di-tetra-butyl-ammoniumsalz (0.5) | 2,4,6-Trimethyl-m-phenylendiamin (9.5) | Isophthal-säure-di-chlorid (9.0) | Diethylmalon-säuredichlorid (1.0) | 80 | 3,5 | 0.167 |
| A10 | 4,4'-Diamino-trans-stilben-2,2'-disul-fonsäure-di-tetra-butylammoniumsalz (0.5) | 3,3'-5,5'-Tetra-methylbenzidin (9.5) | Isophthalsäure-dichlorid (3.0) | Terephthal-säuredichlorid (7.0) | 80 | 3,5 | 1,257 |
| A11[4] | 3,5-Diamino-2,4,6-trimethylbenzol-1-sulfonsäure-tetra-butylammoniumsalz (8.0) | 2,4,6-Trimethyl-m-phenylendiamin (1.0) | Isophthalsäure-dichlorid (3.0) | Terephthal-säuredichlorid (7.0) | 80 | 3,5 | 0,460 |

Tabelle 1 (Fortsetzung)

| Bsp. Nr. | Diamin 1 (mMol) | Diamin 2 (mMol) | 1. Dicarbonsäuredichlorid (mMol) | 2. Dicarbonsäuredichlorid (mMol) | NMP [1] (Menge in g) | PPO [2] (Menge in g) | Inhärente [3] Viskosität (dl/g) |
|---|---|---|---|---|---|---|---|
| A12 | 3,5-Diamino-2,4,6-trimethylbenzol-1-sulfonsäure-tetrabutylammoniumsalz (1.0) | 2,4,6-Trimethyl-m-phenylendiamin (9.0) | Isophthalsäuredichlorid (9.0) | trans-3,6-Endomethylen-1,2,3,6-tetrahydrophthaloyldichlorid (1.0) | 80 | 3,5 | 0.249 |
| A13 | 3,5-Diamino-2,4,6-trimethylbenzol-1-sulfonsäure-tetrabutylammoniumsalz (1.0) | 2,4,6-Trimethyl-m-phenylendiamin (9.0) | Terephthalsäuredichlorid (7.0) | trans-3,6-Endomethylen-1,2,3,6-tetrahydrophthaloyldichlorid (3.0) | 80 | 3,5 | 0.246 |
| A14 [5] | 4,4'-Diamino-trans-stilben-2,2'-disulfonsäure-di-tetrabutylammoniumsalz (0.5) | 4,4'-Diamino-3,3'-diethyl-5,5'-dimethyl-diphenylmethan (9.5) | Isophthalsäuredichlorid (2.9) | 4,4'-Stilbendicarbonyldichlorid (0.5) | 80 | 3,5 | 0,249 |

Tabelle 1 (Fortsetzung)

| Bsp. Nr. | Diamin 1 (mMol) | Diamin 2 (mMol) | 1. Dicarbon-säuredichlorid (mMol) | 2. Dicarbon-säuredichlorid (mMol) | NMP 1) (Menge in g) | PPO 2) (Menge in g) | Inhärente 3) Viskosität (dl/g) |
|---|---|---|---|---|---|---|---|
| A15 | 4,4'-Diamino-trans-stilben-2,2'-disulfon-säure-di-tetra-butylammoniumsalz (0.5) | 4,4'-Diamino-3,3'-diethyl-5,5'-di-methyl-diphenyl-methan (9.5) | Tetrachloro-terephthaloyl-dichlorid (3.0) | Terephthal-säuredichlorid (7.0) | 80 | 3,5 | 0.192 |

1) NMP: N-Methylpyrrolidon

2) PPO: Propylenoxid

3) Inhärente Viskosität: 0,5 Gew.-% in NMP, 25°C

4) 3. Diamin: 1,0 mMol 2,4-Diaminobenzoesäure

5) 3. Dicarbonsäuredichlorid: 6,6 mMol Terephthaloyldichlorid

B) Permeationsversuche

Beispiele B1 bis B8:

In der Mitte einer Druckzelle wird die Membran (Durchmesser 4,7 cm, Dicke 25 μm) in der Halterung befestigt. Jede Kammer ist mit einem Reservoir für die Lösung bzw. das reine Lösungsmittel verbunden, an die eine Pumpe angeschlossen ist. Zwischen Reservoirs und Pumpen befinden sich Leitfähigkeitszellen und zwischen Auslauf und Reservoirs befinden sich UV-Detektoren. Auf der Seite mit der Lösung wird ein Druck von 2,5 MPa und auf der Seite mit dem Lösungsmittel ein Druck von 0,3 MPa eingestellt und die Lösung sowie das Lösungsmittel in gleicher Richtung zirkuliert. Die Detektoren messen kontinuierlich die Leitfähigkeit und UV-Absorption. Die Auswertung der Messdaten erfolgt mittels eines Computerprogramms. Die Detektoren werden jeweils mit der organischen Verbindung (Zimtsäuremethylester) und dem Salz der Carbonsäure (Lithiumsalz der Zimtsäure) geeicht und die Eichkurven in Algorithmen umgewandelt, aus denen die gewünschten Daten errechnet werden. Als Lösungsmittel wird Methanol verwendet und die UV-Detektion wird bei 305 nm vorgenommen. Die Temperatur beträgt 25°C.

Die Selektivität S ist wie folgt definiert, wobei $G^p$ Gewichtsanteil im Permeat und $G^L$ Gewichtsanteil in der Lösung bedeutet:

$$S = \frac{G^P\text{ (unpolarere organische Verbindung)}/G^P\text{ (Salz der Carbonsäure)}}{G^L\text{ (unpolarere organische Verbindung)}/G^L\text{ (Salz der Carbonsäure)}}$$

Weitere Angaben sind in der nachfolgenden Tabelle 2 enthalten.

Tabelle 2

| Bsp. Nr. | Copolyamid von Beispiel | Permeationsgeschwindigkeit (mg · $min^{-1}$ · $cm^{-2}$) | Mengen am Versuchsbeginn [1] | | Versuchsdauer (min) | permeierte Mengen am Versuchsende | | Selektivität |
|---|---|---|---|---|---|---|---|---|
| | | | Zimtsäuremethylester (mg) | Li-Cinnamat (mg) | | Zimtsäuremethylester (mg) | Li-Cinnamat (mg) | |
| B1 | A1 | 0,285 | 25 | 25 | 3060 | 1,00 | 0,25 | 3,95 |
| B2 | A2 | 0,339 | 25 | 25 | 3060 | 0,94 | 0,11 | 8,52 |
| B3 | A4 | 1,766 | 25 | 25 | 1680 | 3,25 | 1,17 | 2,78 |
| B4 | A5 | 0,273 | 25 | 25 | 3780 | 0,96 | 0,50 | 1,11 |
| B5 | A6 | 0,160 | 25 | 25 | 5040 | 0,23 | 0,10 | 2,28 |
| B6 | A7 | 2,174 | 25 | 25 | 1020 | 2,05 | 0,24 | 8,54 |
| B7 | A8 | 0,057 | 25 | 25 | 7050 | 0,48 | 0,27 | 1,77 |
| | | 0,057 | 75 [2] | 75 [3] | 2520 | 1,98 [2] | 0,11 [3] | 18,00 |
| B8 | A10 | 0,446 | 75 [2] | 75 [3] | 2040 | 7,23 [2] | 0,22 [3] | 32,86 |

[1] 0,005 Gew.-%

[2] Dimethylphthalat

[3] 35 % Trimellitsäure-tri-Li-Salz und 65 % Trimellitsäure-di-Li-Salz

C) Belichtungsbeispiel

Beispiel C1:

Ein Copolyamid aus 13,5 Gewichtsteilen 4,4′-Diamino-3,3′,5,5′-tetramethyldiphenylmethan, 1,5 Gewichtsteilen 3,5-Diamino-2,4,6-trimethylbenzol-1-sulfonsäure-tetra-n-butylammoniumsalz, 7,5 Gewichtsteilen Terephthalsäuredichlorid und 7,5 Teilen 4-Nitroisophthalsäuredichlorid wird in N-Methylpyrrolidon gelöst (10 Gew.-%) und damit mit einem 12 μ Kabel ein Kupferlaminat beschichtet (Spin-coating) und bei 100°C getrocknet. Danach belichtet man unter einer Photomaske (Stouffer-Keil) während 180 Sekunden mit einer Leistung von 40 mW/cm² (Lampe: Ultralux 5000 H, Distanz 70 cm). Danach wird mit wässriger 2n NaOH entwickelt. Die Stoufferstufe 6 ist noch gut abgebildet.

## Patentansprüche

1. Verfahren zum Anreichern oder Trennen von Salzen organischer Carbonsäuren von nicht salzartigen organischen Verbindungen mit einer semipermeablen Membran, dadurch gekennzeichnet, dass man eine Lösung der Salze und organischen Verbindungen in einem unsubstituierten oder mit $C_1$-$C_3$-Alkoxy substituierten $C_1$-$C_4$-Alkanol, Mischungen solcher Alkanole oder Mischungen solcher Alkanole mit Ethern mit einer Seite der Membran in Berührung bringt, sich auf der Gegenseite der Membran das reine Lösungsmittel befindet, und die Membran aus einem Copolyamid oder Copolyimidamid aus (a) mindestens einem aromatischen Dicarbonsäurerest mit 8 bis 20 C-Atomen und/oder (b) mindestens einem trivalenten aromatischen Tricarbonsäurerest mit 9 bis 20 C-Atomen, die, unsubstituiert oder mit Halogen, Nitro, $C_1$-$C_4$-Alkyl oder $C_1$-$C_4$-Alkoxy substituiert sind, (c) mindestens einem ersten divalenten und/oder trivalenten ein- oder zweikernigen aromatischen Diaminrest, und (d) mindestens einem zweiten divalenten und/oder trivalenten und mindestens eine $-SO_3M$-Gruppe enthaltenden aromatischen ein- oder zweikernigen Diaminrest, die je 6 bis 18 C-Atome enthalten und je unsubstituiert oder mit Halogen oder $C_1$-$C_4$-Alkyl substituiert sind, und M $H^{\oplus}$, ein ein- bis dreiwertiges Metallkation, $NH_4^{\oplus}$ oder ein organisches Ammoniumkation mit 1 bis 30 C-Atomen bedeutet.

2. Verfahren gemäss Anspruch 1, dadurch gekennzeichnet, dass die Konzentration der Salze und organischen Verbindungen 0,0001 bis 10 Gew.-% beträgt, bezogen auf die Lösung.

3. Verfahren gemäss Anspruch 1, dadurch gekennzeichnet, dass die Membran eine Dicke von 5 bis 300 μm aufweist.

4. Verfahren gemäss Anspruch 1, dadurch gekennzeichnet, dass das Lösungsmittel Methanol, Ethanol, 1- oder 2-Propanol oder 2-Methoxyethanol ist.

5. Verfahren gemäss Anspruch 1, dadurch gekennzeichnet, dass es sich bei den Salzen der organischen Carbonsäure um Ammonium- oder Metallsalze handelt.

6. Verfahren gemäss Anspruch 1, dadurch gekennzeichnet, dass es sich bei der organischen Carbonsäure um eine aliphatische, cycloaliphatische, aromatische, heterocyclische oder heteroaromatische Monocarbonsäure mit 1 bis 18 C-Atomen handelt.

7. Verfahren gemäss Anspruch 1, dadurch gekennzeichnet, dass die nicht salzartige organische Verbindung ein Ester einer organischen Carbonsäure mit insgesamt 2 bis 16 C-Atomen, ein Ether mit 2 bis 12 C-Atomen, ein Keton mit 3 bis 16 C-Atomen oder ein Alkohol mit 5 bis 16 C-Atomen ist.

8. Verfahren gemäss Anspruch 1, dadurch gekennzeichnet, dass es bei Raumtemperatur durchgeführt wird.

9. Verfahren gemäss Anspruch 1, dadurch gekennzeichnet, dass auf der Seite der Lösung ein Druck von 1 MPa bis 10 MPa eingestellt wird.

10. Verfahren gemäss Anspruch 1, dadurch gekennzeichnet, dass es im Gegenstrom durchgeführt wird.

11. Verfahren gemäss Anspruch 1, dadurch gekennzeichnet, dass das Copolyamid oder Copolyimidamid 60

bis 95 Mol-% Diaminreste (c) und 5 bis 40 Mol-% Diaminreste (d) enthält, bezogen auf die Diaminreste.

**12.** Verfahren gemäss Anspruch 11, dadurch gekennzeichnet, dass 75 bis 95 Mol-% Diaminreste (c) und 5 bis 25 Mol-% Diaminreste (d) enthalten sind.

**13.** Verfahren gemäss Anspruch 1, dadurch gekennzeichnet, dass das Copolyamid oder Copolyimidamid oder deren Mischpolymere eine inhärente Viskosität von 0,2 bis 3,0 dl/g aufweist, gemessen bei 25°C in einer Lösung von 0,5 Gew.-% dieser Copolymeren in N-Methylpyrrolidon.

**14.** Verfahren gemäss Anspruch 1, dadurch gekennzeichnet, dass M für $H^{\oplus}$, $NH_4^{\oplus}$, ein Alkalimetallkation oder ein primäres, sekundäres, tertiäres oder quaternäres Ammoniumkation mit 1 bis 24 C-Atomen steht.

**15.** Verfahren gemäss Anspruch 1, dadurch gekennzeichnet, dass das Copolyamid und/oder Copolyimidamid
a) 60 bis 95 Mol-% mindestens eines wiederkehrenden Strukturelementes der Formeln I und/oder Ia,

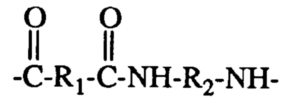

(I),

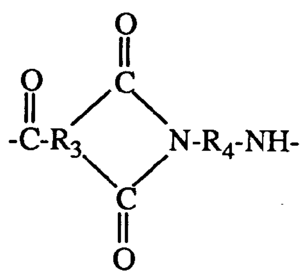

(Ia),

und
b) 5 bis 40 Mol-% mindestens eines wiederkehrenden Strukturelementes der Formeln II und/oder IIa,

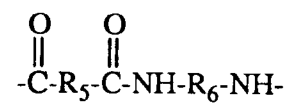

(II),

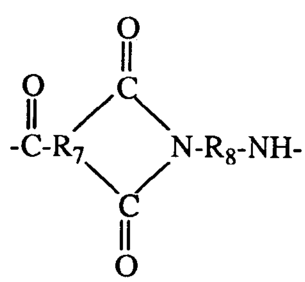

(IIa)

enthalten, bezogen auf die Copolymeren, worin $R_1$ und $R_5$ unabhängig voneinander einen unsubstituierten oder mit Cl, Br, $NO_2$, $C_1$-$C_4$-Alkyl oder $C_1$-$C_4$-Alkoxy substituierten Rest der Formeln

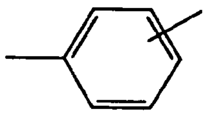 , 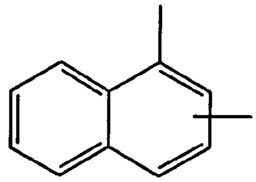 , 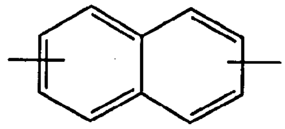 oder

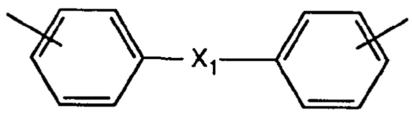

darstellen, worin $X_1$ für eine direkte Bindung, $-CH_2-$,

$-(CH_2)_2-$, -HC=CH-, $CH_3CH<$, $(CH_3)_2C<$,

$-NR_9-$, -N=N-, -CO-, -O-, -S-, -SO- oder $-SO_2-$ steht, und $R_9$ H oder $C_1$-$C_6$-Alkyl bedeutet, $R_3$ und $R_7$ unabhängig voneinander einen Rest der Formeln

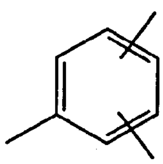 , 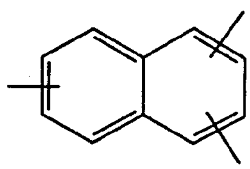 , 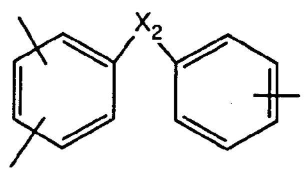

darstellen, worin zwei Bindungen an zwei benachbarte C-Atome gebunden sind, und $X_2$ unabhängig die gleiche Bedeutung wie $X_1$ hat, $R_2$ und $R_4$ unabhängig voneinander einen unsubstituierten oder mit Cl, Br oder $C_1$-$C_4$Alkyl substituierten Rest der Formeln

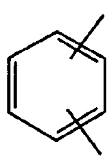 , 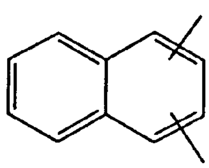 , 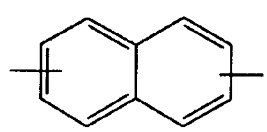 , 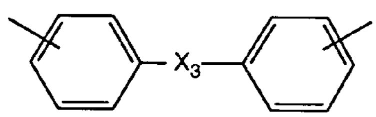

bedeuten, worin $X_3$ unabhängig voneinander die gleiche Bedeutung wie $X_1$ hat, und $R_6$ und $R_8$ unabhängig voneinander einen Rest der Formeln

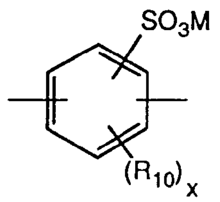
,
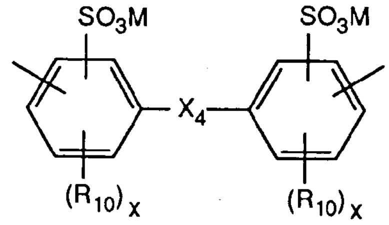

darstellen, worin $X_4$ unabhängig die gleiche Bedeutung wie $X_1$ hat, $R_{10}$ für $C_1$-$C_4$-Alkyl steht, x für 0, 1, 2 oder 3 steht und M $H^{\oplus}$, $NH_4^{\oplus}$, ein Alkalimetallkation oder ein primäres, sekundäres, tertiäres oder quaternäres Ammoniumkation mit 1 bis 24 C-Atomen darstellt.

**16.** Verfahren gemäss Anspruch 15, dadurch gekennzeichnet, dass es sich um ein Copolyamid mit Strukturelementen der Formeln I und II handelt, und $X_1$ und $X_4$ unabhängig voneinander eine direkte Bindung, $-CH_2-$ , -CO- , -O- oder -S- bedeuten.

**17.** Verfahren gemäss Anspruch 15, dadurch gekennzeichnet, dass in den Strukturelementen der Formeln I und II des Copolyamids $R_1$ und $R_5$ unabhängig voneinander für unsubstituiertes oder mit $-NO_2-$, -Cl oder -Br substituiertes m- und/oder p-Phenylen steht, $R_2$ unsubstituiertes oder mit -Cl, -Br, Methyl und/oder Ethyl substituiertes Phenylen oder in beiden o-Stellungen zu den Aminogruppen mit Methyl und/oder Ethyl substituiertes 3,3'- oder 4,4'-Diphenylmethandiyl bedeutet, und $R_6$ einen Rest der Formeln

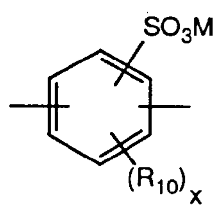
,
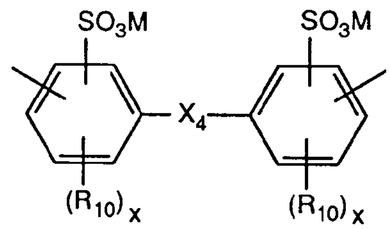

darstellt, worin $X_4$ eine direkte Bindung oder $-CH_2-$ bedeutet, $R_{10}$ für Methyl oder Ethyl steht, x für 1, 2 oder 3 steht, und M $H^{\oplus}$, $NH_4^{\oplus}$, $Li^{\oplus}$, $Na^{\oplus}$, $K^{\oplus}$, $Rb^{\oplus}$, $Cs^{\oplus}$ oder quaternäres Ammonium mit 4 bis 16 C-Atomen bedeutet.

**18.** Verfahren gemäss Anspruch 17, dadurch gekennzeichnet, dass $R_6$ ein Rest der Formel

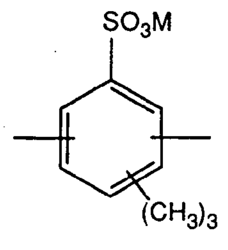

ist, worin M $H^{\oplus}$, $NH_4^{\oplus}$, $Li^{\oplus}$, $Na^{\oplus}$, $K^{\oplus}$, $Rb^{\oplus}$, $Cs^{\oplus}$ oder quaternäres Ammonium mit 4 bis 16 C-Atomen bedeutet.

**19.** Copolymere aus der Gruppe der Copolyamide und Copolyimidamide mit einer inhärenten Viskosität von 0,2 bis 3,0 dl/g, gemessen bei 25°C in einer Lösung von 0,5 Gew.-% Copolymer in N-Methylpyrrolidon, die enthalten, bezogen auf das Copolymer:

a) 10 bis 95 Mol-% mindestens eines wiederkehrenden Strukturelements der Formeln III und/oder IIIa

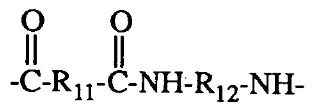

(III),

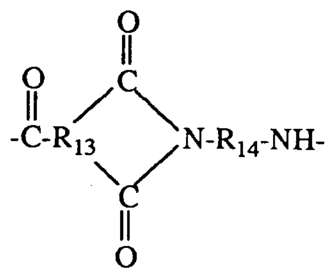

(IIIa),

und

b) 5 bis 90 Mol-% mindestens eines wiederkehrenden Strukturelements der Formeln IV und/oder IVa

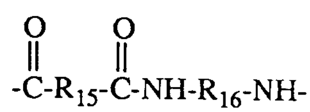

(IV),

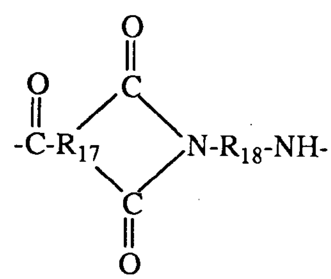

(IVa)

worin

$R_{11}$ und $R_{15}$ unabhängig voneinander der divalente Rest einer ein- oder zweikernigen aromatischen Dicarbonsäure mit 8 bis 20 C-Atomen bedeuten, die je unsubstituiert oder mit Halogen, Nitro, $C_1$-$C_4$-Alkyl oder $C_1$-$C_4$-Alkoxy substituiert sind,

$R_{13}$ und $R_{17}$ unabhängig voneinander der trivalente Rest einer ein- oder zweikernigen aromatischen Tricarbonsäure mit 9 bis 20 C-Atomen darstellen, die je unsubstituiert oder mit Halogen oder Nitro substituiert sind,

$R_{12}$ und $R_{14}$ unabhängig voneinander einen Rest der Formeln

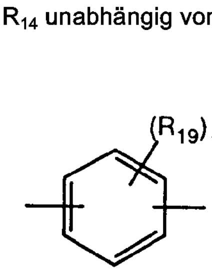

oder

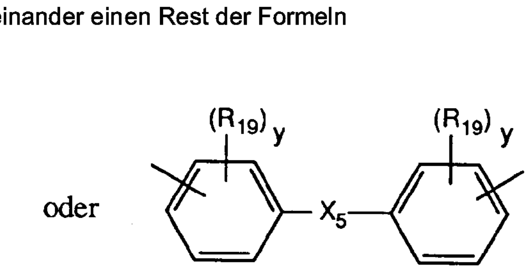

darstellen,

worin $R_{19}$ $C_1$-$C_4$-Alkyl bedeutet, $X_5$ eine direkte Bindung, $-CH_2-$,

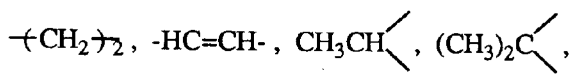

$-NR_9-$ , -N=N- , -CO- , -O- oder -S- darstellt und $R_9$ für H oder $C_1$-$C_6$-Alkyl steht, y für 0, 1, 2, 3 oder 4 steht und

$R_{16}$ und $R_{18}$ unabhängig voneinander einen Rest der Formeln

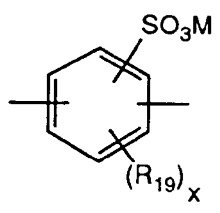

oder

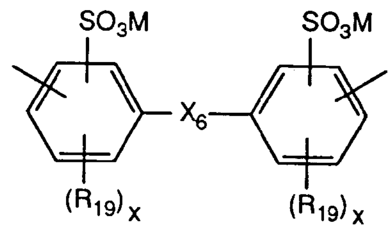

darstellen, worin $R_{19}$ $C_1$-$C_4$-Alkyl bedeutet, x für 0, 1, 2 oder 3 steht, $X_6$ eine direkte Bindung, $-CH_2-$,

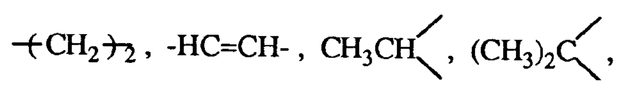

$-NR_9-$ , -N=N- , -CO-, -O-, -S-, -SO- oder $-SO_2-$ bedeutet und $R_9$ für H oder $C_1$-$C_6$-Alkyl steht, und M $H^{\oplus}$, $NH_4^{\oplus}$, ein einwertiges Metallkation oder ein organisches Ammoniumkation mit 1 bis 30 C-Atomen bedeutet,

mit der Massgabe, dass in mindestens einem der Reste $R_{12}$, $R_{14}$, $R_{16}$ und $R_{18}$ mindestens zwei Reste $R_{19}$ in den Orthostellungen der freien Bindungen gebunden sind.

**20.** Copolymere gemäss Anspruch 19, dadurch gekennzeichnet, dass $R_{11}$ und $R_{15}$ unabhängig voneinander einen unsubstituierten oder mit Cl, Br oder $NO_2$ substituierten Rest der Formeln

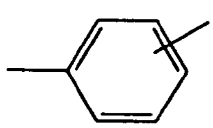

,

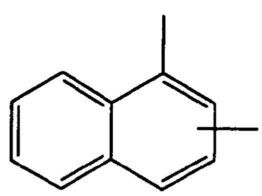

,

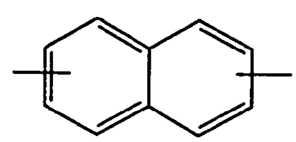

oder

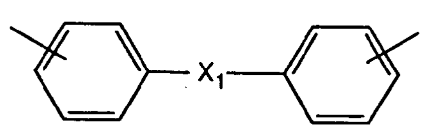

darstellen, worin $X_1$ für eine direkte Bindung, $-CH_2-$,

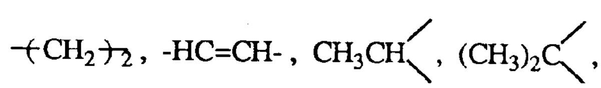

$-NR_9-$, -N=N-, -CO-, -O-, -S-, -SO- oder $-SO_2-$ steht, und $R_9$ H oder $C_1$-$C_6$-Alkyl bedeutet, und $R_{13}$ und $R_{17}$ unabhängig voneinander einen Rest der Formeln

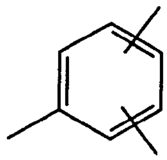 , 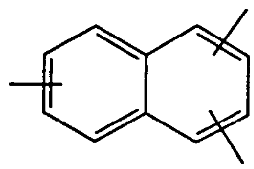 , 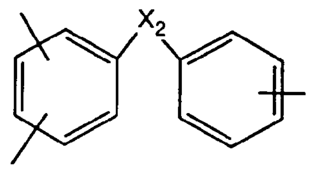

darstellen, worin zwei Bindungen an zwei benachbarte C-Atome gebunden sind, und $X_2$ unabhängig die gleiche Bedeutung wie $X_1$ hat.

**21.** Copolymere gemäss Anspruch 19, dadurch gekennzeichnet, dass $R_{11}$ und $R_{15}$ unabhängig voneinander unsubstituiertes oder mit Cl, Br oder $NO_2$ substituiertes o-, m- oder p-Phenylen oder einen Rest der Formel

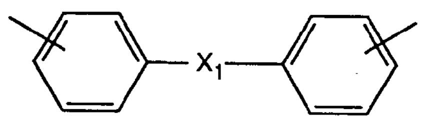

darstellen, worin die freien Bindungen in m- oder p-Stellung zur Gruppe $X_1$ gebunden sind und $X_1$ eine direkte Bindung, $-CH_2-$, -O-, -S-, $-SO_2-$ oder -CO- bedeutet.

**22.** Copolymere gemäss Anspruch 21, dadurch gekennzeichnet, dass $X_1$ für -CO- steht.

**23.** Copolymere gemäss Anspruch 19, dadurch gekennzeichnet, dass M für $H^{\oplus}$, $NH_4^{\oplus}$, ein Alkalimetallkation oder ein primäres, sekundäres, tertiäres oder quaternäres Ammoniumkation mit 1 bis 24 C-Atomen darstellt.

**24.** Copolymere gemäss Anspruch 19, dadurch gekennzeichnet, dass $R_{19}$ für Methyl oder Ethyl steht.

**25.** Copolymere gemäss Anspruch 19, dadurch gekennzeichnet, dass $R_{12}$ und $R_{14}$ unabhängig voneinander einen Rest der Formeln

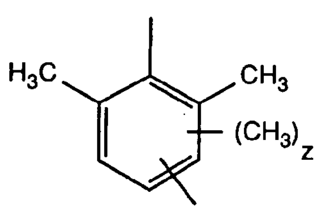 oder 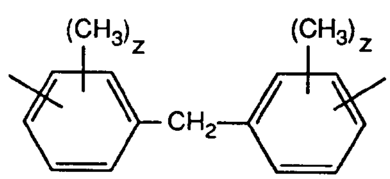

darstellen,
worin in der ersten Formel z 0, 1 oder 2 ist und die freien Bindungen in m- oder p-Stellung zueinander stehen, und in der zweiten Formel die freien Bindungen in m- oder p-Stellung zur $CH_2$-Gruppe gebunden sind, und die beiden $CH_3$-Gruppen je in den Orthostellungen der freien Bindungen gebunden sind.

**26.** Copolymere gemäss Anspruch 19, dadurch gekennzeichnet, dass $R_{16}$ und $R_{18}$ unabhängig voneinander einen Rest der Formeln

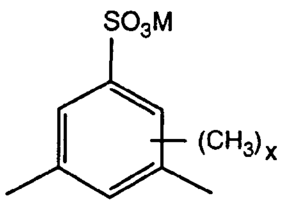 oder 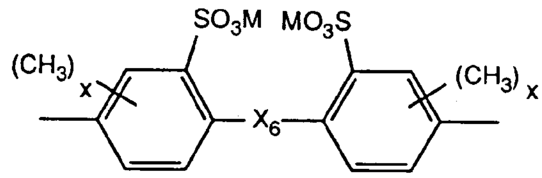

darstellen, worin x 0, 1, 2 oder 3 ist, $X_6$ eine direkte Bindung, $-CH_2-$, -O-, -S-, -CO- oder $-SO_2-$ bedeutet und M für $H^{\oplus}$, $NH_4^{\oplus}$, ein Alkalimetallkation oder primäres, sekundäres, tertiäres oder quaternäres Ammonium mit 1 bis 24 C-Atomen steht.

**27.** Copolymere gemäss Anspruch 19, dadurch gekennzeichnet, dass es ein Copolyamid mit wiederkehrenden Strukturelementen der Formeln

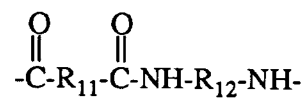 und

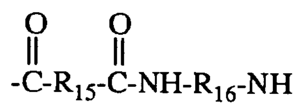

ist,
worin $R_{11}$ und $R_{15}$ unabhängig voneinander unsubstituiertes oder mit Cl, Br oder Nitro substituiertes m- oder p-Phenylen oder

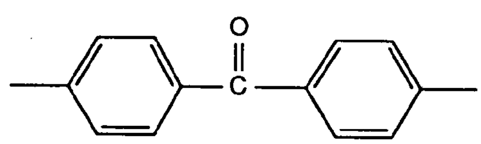

bedeutet,
$R_{12}$ für einen Rest der Formeln

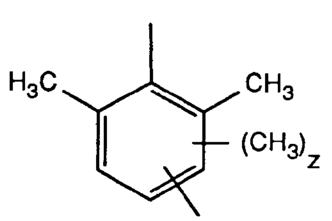 oder 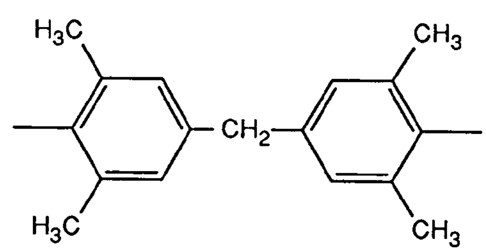

steht, worin z 0, 1 oder 2 ist, und
$R_{16}$ einen Rest der Formel

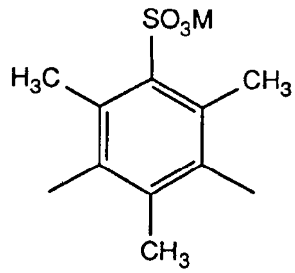

darstellt, worin M für $H^{\oplus}$, $NH_4^{\oplus}$, ein Alkalimetallkation oder primäres, sekundäres, tertiäres oder quaternäres Ammonium mit 1 bis 24 C-Atomen steht.

**28.** Copolymere gemäss Anspruch 19, dadurch gekennzeichnet, dass sie 60 bis 95 Mol-% Strukturelemente der Formeln III und/oder IIIa und 5 bis 40 Mol-% Strukturelemente der Formeln IV oder IVa enthalten.

**29.** Verfahren zur Herstellung von Copolymeren gemäss Anspruch 19, dadurch gekennzeichnet, dass man, bezogen auf das Copolymer,

a) 50 Mol-% mindestens einer Dicarbonsäure der Formel V und/oder Va

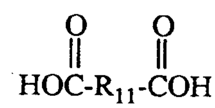 (V)

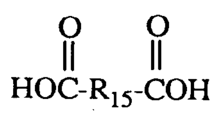 (Va)

und/oder mindestens einer Tricarbonsäure der Formeln VI und/oder VIa

$$R_{13}\text{-}(COOH)_3 \quad (VI)$$

$$R_{17}\text{-}(COOH)_3 \quad (VIa)$$

oder deren Polyamid bildenden Derivate mit

b) 5 bis 47,5 Mol-% mindestens eines Diamins der Formeln VII und/oder VIIa

$$H_2N\text{-}R_{12}\text{-}NH_2 \quad (VII)$$

$$H_2N\text{-}R_{14}\text{-}NH_2 \quad (VIIa)$$

und

c) 2,5 bis 45 Mol-% mindestens eines Diamins der Formeln VIII und/oder VIIIa

$$H_2N\text{-}R_{16}\text{-}NH_2 \quad (VIII)$$

$$H_2N\text{-}R_{18}\text{-}NH_2 \quad (VIIIa)$$

umsetzt.

**30.** Beschichtetes Material, enthaltend auf einem Träger eine Schicht eines Copolymers gemäss Anspruch 19, wobei $X_5$ zusätzlich -SO- oder $-SO_2-$ bedeuten kann.

**31.** Verfahren zur Herstellung von Schutzschichten oder Reliefabbildungen, dadurch gekennzeichnet, dass man die Schicht des Materials gemäss Anspruch 30 flächenmässig bestrahlt, oder unter einer Photomaske bestrahlt und danach in einem wässrig-basischen Medium durch Lösen der unbestrahlten Anteile entwickelt.